# EUROPEAN PATENT SPECIFICATION

(11) **EP 2 087 095 B1**
(45) Date of publication and mention of the grant of the patent: **16.07.2014**
(21) Application number: 07819811.6
(22) Date of filing: 14.11.2007
(51) Int. Cl.: C12N 1/20, C12R 1/01, A61K 39/106, C12Q 1/04

(54) **IN VITRO CULTIVATION OF HELICOBACTER SPECIES**
IN-VITRO-KULTIVIERUNG VON HELICOBACTER-SPEZIES
CULTURE IN VITRO DE L'ESPECES D'HELICOBACTER

(30) Priority: 14.11.2006 US 865723 P
(43) Date of publication of application: 12.08.2009
(73) Proprietor: Universiteit Gent, 9000 Gent (BE)
(72) Inventor: BAELE, Margot, 9000 Gent (BE); HAESEBROUCK, Freddy, 8301 Knokke-Heist (BE)
(74) Representative: Bird, William Edward
(86) International application number: PCT/EP2007/009855
(87) International publication number: WO 2008/058727

(56) References cited:
- EP-A- 0 540 897
- EP-A- 1 035 219
- WO-A-00/75285
- WO-A-97/13527
- WO-A-2006/091721
- WO-A-2006/133879
- US-A1- 2001 055 787
- VAN DEN BULCK K ET AL: "Helicobacter cynogastricus sp nov., isolated from the canine gastric mucosa" INTERNATIONAL JOURNAL OF SYSTEMATIC AND EVOLUTIONARY MICROBIOLOGY, vol. 56, no. Part 7, July 2006 (2006-07), pages 1559-1564, XP002475180 ISSN: 1466-5026
- ANDERSEN L P ET AL: "Characterization of a culturable "Gastrospirillum hominis" (Helicobacter heilmannii) strain isolated from human gastric mucosa" JOURNAL OF CLINICAL MICROBIOLOGY, vol. 37, no. 4, April 1999 (1999-04), pages 1069-1076, XP002475181 ISSN: 0095-1137
- HELLEMANS A ET AL: "Evaluation of antibiotic treatment against "Candidatus Helicobacter suis" in a mouse model" ANTIMICROBIAL AGENTS AND CHEMOTHERAPY, vol. 49, no. 11, November 2005 (2005-11), pages 4530-4535, XP002475182 ISSN: 0066-4804
- HELLEMANS ET AL: "Protective immunization against ''Candidatus Helicobacter suis'' with heterologous antigens of H. pylori and H. felis" VACCINE, vol. 24, no. 14, 24 March 2006 (2006-03-24), pages 2469-2476, XP005309253 ISSN: 0264-410X
- DE GROOTE DOMINIC ET AL: "'Candidatus Helicobacter suis', a gastric helicobacter from pigs, and its phylogenetic relatedness to other gastrospirilla" INTERNATIONAL JOURNAL OF SYSTEMATIC BACTERIOLOGY, vol. 49, no. 4, October 1999 (1999-10), pages 1769-1777, XP002475183 ISSN: 0020-7713 cited in the application
- DIETERICH C ET AL: "Urease-based mucosal immunization against Helicobacter heilmannii infection induces corpus atrophy in mice" INFECTION AND IMMUNITY, vol. 67, no. 11, November 1999 (1999-11), pages 6206-6209, XP002408158 ISSN: 0019-9567
- GROOTE DE D ET AL: "Detection of Candidatus Helicobacter suis in gastric samples of pigs by PCR: Comparison with other invasive diagnostic techniques" JOURNAL OF CLINICAL MICROBIOLOGY, vol. 38, no. 3, March 2000 (2000-03), pages 1131-1135, XP002408281 ISSN: 0095-1137
- O'ROURKE JANI L ET AL: "Description of 'Candidatus Helicobacter heilmannii' based on DNA sequence analysis of 16S rRNA and urease genes" INTERNATIONAL JOURNAL OF SYSTEMATIC AND EVOLUTIONARY MICROBIOLOGY, vol. 54, no. Part 6, November 2004 (2004-11), pages 2203-2211, XP002475424 ISSN: 1466-5026 cited in the application
- SOLNICK J V ET AL: "Emergence of diverse Helicobacter species in the pathogenesis of gastric and enterohepatic diseases" CLINICAL MICROBIOLOGY REVIEWS, vol. 14, no. 1, January 2001 (2001-01), pages 59-97, XP002475184 ISSN: 0893-8512
- BAELE M ET AL: "Isolation and characterization of Helicobacter suis sp nov from pig stomachs" ZOONOSES PUBLIC HEALTH, vol. 54, no. Suppl. 1, 2007, pages 6-7, XP008090039 & 14TH INTERNATIONAL WORKSHOP ON CAMPYLOBACTER, HELICOBACTER AND RELATED ORGANISMS; ROTTERDAM, NETHERLANDS; SEPTEMBER 02 -05, 2007 ISSN: 1863-1959
- BAELE M ET AL: "Helicobacter baculiformis sp. nov., isolated from feline stomach mucosa." INTERNATIONAL JOURNAL OF SYSTEMATIC AND EVOLUTIONARY MICROBIOLOGY, vol. 58, no. Pt 2, February 2008 (2008-02), pages 357-364, XP002475792 ISSN: 1466-5026
- M. BAELE ET AL: 'Isolation and characterization of Helicobacter suis sp. nov. from pig stomachs' INTERNATIONAL JOURNAL OF SYSTEMATIC AND EVOLUTIONARY MICROBIOLOGY vol. 58, no. 6, 01 June 2008, pages 1350 - 1358, XP055020021 DOI: 10.1099/ijs.0.65133-0 ISSN: 1466-5026
- PICKERING ET AL: 'Bacteriologic and serologic findings in experimental pyelonephritis caused by Ureaplasma urealyticum.' INFECTION AND IMMUNITY vol. 57, no. 4, 01 April 1989, pages 1235 - 1239, XP055021396 ISSN: 0019-9567

### SEQUENCE LISTING

<110> Universiteit Gent Baele, Margo Haesebrouck, Freddy
<120> In vitro cultivation of Helicobacter species
<130> R4479-PCT
<150> US 60/865,723
   <151> 2006-11-14
<160> 7
<170> PatentIn version 3.3
<210> 1
   <211> 18
   <212> DNA
   <213> Artificial sequence
<220>
   <223> primer
<400> 1
   tcaaactagg accgagtc 18
<210> 2
   <211> 20
   <212> DNA
   <213> Artificial sequence
<220>
   <223> primer
<400> 2
   taccttgtta cttcacccca 20
<210> 3
   <211> 18
   <212> DNA
   <213> Artificial sequence
<220>
   <223> primer
<400> 3
   cagcagccgc ggtaatac 18
<210> 4
   <211> 20
   <212> DNA
   <213> Artificial sequence
<220>
   <223> primer
<400> 4
   ctcctacggg aggcagcagt 20
<210> 5
   <211> 20
   <212> DNA
   <213> Artificial sequence
<220>
   <223> primer
<400> 5
   gttgcgctcg ttgcgggact 20
<210> 6
   <211> 19
   <212> DNA
   <213> Artificial sequence
<220>
   <223> primer
<400> 6
   aactcaaagg aattgacgg 19
<210> 7
   <211> 1421
   <212> DNA
   <213> Helicobacter sp.
<220>
   <221> misc_feature
   <222> (1)..(1421)
   <223> Helicobacter suis 16S ribosomal RNA gene, partial sequence Genbank
   Accession AF127028
<400> 7
   tgcaagtcga acgatgaagc ctagcttgct aggttgatta gtggcgcacg ggtgagtaat     60
   gcatagatga catgcccttt agtttggaat agccactaga aatggtgatt aataccaaat    120
   actaccttac gagggaaaga tttatcgcta aaggattggt ctatgtccta tcagcttgtt    180
   ggtgaggtaa aggctcacca aggctatgac gggtatccgg cctgagaggg tgagcggaca    240
   cactggaact gagacacggt ccagactcct acgggaggca gcagtaggga atattgctca    300
   atgggggaaa ccctgaagca gcaacgccgc gtggaggatg aaggttttag gatcgtaaac    360
   tccttttgtt agagaagata atgacggtat ctaacgaata agcaccggct aactccgtgc    420
   cagcagccgc ggtaatacgg agggtgcaag cgttactcgg aatcactggg cgtaaagagt    480
   gcgtaggcgg gaggacaagt caggtgtgaa atcctatggc ttaaccatag aactgcattt    540
   gaaactatcc ttctggagtg tgggagaggt aggtggaatt cttggtgtag gggtaaaatc    600
   cgtagagatc aagaggaata ctcattgcga aggcgacctg ctggaacatc actgacgctg    660
   attgcacgaa agcgtgggga gcaaacagga ttagataccc tggtagtcca cgccctaaac    720
   gatggatgct agttgttggg aggctttgtc tttccagtaa tgcagctaac gccttaagca    780
   tcccgcctgg ggagtacggt cgcaagatta aaactcaaag gaatagacgg ggacccgcac    840
   aagcggtgga gcatgtggtt taattcgaag ttacacgaag aaccttacct aggcttgaca    900
   ttgaaggaat tccctagaaa taggggagtg tctagcttgc tagaccctga aaacaggtgc     960
   tgcacggctg tcgtcagctc gtgtcgtgag atgttgggtt aagtcccgca acgagcgcaa   1020
   ccctttttct tagttgctaa caggttatgc tgcgcactct aagaagactg cctgcgtaag   1080
   caggaggaag gtgaggacga cgtcaagtca tcatggccct tacgcctagg gctacacacg   1140
   tgctacaatg gggtgcacaa agagatgcaa agccgcgagg cagagctaat ctataaaaca    1200
   cctcctagtt cggattgcag gctgcaactc gcctgcatga agctggaatc gctagtaatc   1260
   gcaaatcagc tatgttgcgg tgaatacgtt cccgggtctt gtactcaccg cccgtcacac   1320
   catgggagtt gtgtttgcct taagtcagga tgctaaagca gctactgccc acggcacaca   1380
   cagcgactgg ggtgaagtcg taacaaggta acccgggcgg c                       1421

## Description

### FIELD OF THE INVENTION

The present invention relates to methods and tools for the *in vitro* isolation and/or cultivation of *Helicobacter* species, more particularly *"Candidatus* Helicobacter suis". The invention further relates to the preparation of antigens and vaccines from isolated cultures of *Helicobacter* species.

### BACKGROUND

*Helicobacter pylori* infections in humans are a major cause of gastric and duodenal ulceration as well as gastric cancer. *H. pylori* is not the only *Helicobacter* species capable of colonising the human gastric mucosa. "*Helicobacter heilmannii*" (proposed name) has been found in approximately 0.96% of gastric biopsies in humans (Heilmann & Borchard (1991) Gut 32, 137-140). This organism is strongly associated with gastritis but is also associated with peptic ulceration, gastric adenocarcinoma, and mucosa-associated lymphoid tissue lymphoma. Recent evidence indicates that *"H. heilmannil'* is not a single species but represents different bacterial species with similar spiral morphologies, most of which are probably of zoonotic origin. Classification into *"H. heilmannii" type* 1 and *"H. heilmannii"* type 2 was established on the basis of 16S rRNA gene sequences (Solnick et al. (1993) J. Infect. Dis. 168, 379-385). More than 50% of the *"H. heilmannii"* infections in humans are due to "*H*. *heilmannii"* type 1 (Trebesius et al. (2001) J. Clin. Microbiol. 39, 1510-1516.). *"H. heilmannii"* type 1 has been shown to be identical to *"Candidatus* H. suis" (O'Rourke et al. (2004) Int. J. Syst. Evol. Microbiol. 54, 2203-2211), a hitherto non-culturable spiral bacterium that colonises the stomachs of more than 60% of slaughter pigs. The actual role of *Candidatus* H. suis in gastric disease in pigs is still a matter of debate, but it has been suggested that this bacterium is associated with gastric ulceration of the *pars oesophagea* and with chronic pyloric gastritis. Mouse inoculation was used to isolate this bacterium from infected pig stomach mucosa (Dick et al. (1989) J. Med. Microbiol. 29, 55-62). Hellemans et al. [(2005) Antimicrob. agents chemother. 49, 4530-4535] modified the existing *in vivo* mouse model of *Candidatus* H. suis infection, for evaluating the antibiotic susceptibility of this organism. *In vitro* cultivation of *Candidatus* H. suis has not been achieved yet.

US2001055787 discloses an agar medium for the growth of *Mycobacterium tuberculosis,* comprising animal serum at a concentration of between about 8% and 12% of the final volume of the agar medium, wherein said medium is at a pH of between about 6.0 to about 6.25.

Pickering et al. (1989) Infect. Immunol. 57,1235-1239, disclose the cultivation of Ureaplasma urealyticum in an agar of pH 5.5, comprising 20 % horse serum. Hellemans et al. (2006) Vaccine 24(14), 2469-2476 relates to cross-immunisation methods against Helicobacter, wherein stomach homogenates comprising Candidatus Helicobacter suis are used to infect animals.

### SUMMARY OF THE INVENTION

The present invention is based on the development of cultivation systems which allow the isolation and cultivation of *Helicobacter* species, more particularly of *"Candidatus* Helicobacter suis", which had not previously been demonstrated to be sustainable as an *in vitro* isolate.

One aspect of the present invention relates to the use of a cultivation recipient comprising a solid cultivation medium, comprising between 7.5% and 15 % blood or between 12.5 and 25 % serum, the solid medium being adjusted to a pH between 5.0 and 5.5, for the isolation and/or cultivation of a *Helicobacter* species.

In a specific embodiment, the solid component comprises nutrients for the growth of fastidious organism. Most particularly these nutrients are selected from the group of Brucella, Mueller-Hinton or Brain Heart Infusion medium.

In a second aspect, the present invention provides methods for the isolation of *Helicobacter* species, more particularly *"Candidatus* Helicobacter suis", from a sample comprising the same *Helicobacter* species, which methods comprise the steps of cultivating the sample comprising *Helicobacter* species in a cultivation system comprising a cultivation medium having a pH between 5.0 and 6.0, which is supplemented with at least 10 % serum or at least 7.0 % blood.

In specific embodiments of these methods, the cultivation medium used comprises nutrients for the growth of fastidious bacteria. More specifically, the nutrients for the growth of fastidious bacteria are selected from the group consisting of Brucella, Mueller-Hinton or Brain Heart Infusion nutrients. Additionally or alternatively, in specific embodiments, the cultivation medium comprises at least one selective substance that inhibits the growth of fungi and/or Gram positive and/or Gram negative micro-organisms other than the *Helicobacter* species to be cultivated. In further specific embodiments, the at least one selective substance is selected from the group consisting of: vancomycin, trimethoprim lactate, polymyxin B, cefsulodin, colistin, amphotericin B, crystal violet, nystatin and nisin.

In specific embodiments of the methods of the present invention described herein, the cultivation medium comprising at least 10% serum comprises between 12.5 and 25 % serum. Alternatively, the cultivation medium comprising at least 7.0% blood comprises between 7.5 and 15 %, more particularly between 10 and 15% blood.

Optionally, the cultivation medium used in the methods of the present invention further comprises one or more growth factors selected from the group of Vitamin B12, L-glutamine, Adenine, Guanine, p-Aminobenzoic acid, L-cystine, NAD (Coenzyme 1), Cocarboxylase, Ferric nitrate, Thiamine and Cysteine hydrochloride.

In a specific embodiment of the methods of the invention described herein, a cultivation system is used which comprises a solid and a liquid component, wherein at least the solid component comprises the cultivation medium as described above. In further embodiments, both the solid and/or liquid component comprise nutrients for the growth of fastidious bacteria. More specifically, these nutrients are selected from the group consisting of Brucella, Mueller-Hinton or Brain Heart Infusion nutrients.

In a further specific embodiment of the methods of the invention, the solid and/or liquid component of the cultivation systems comprise at least one selective substance that inhibits the growth of fungi and/or Gram positive and/or Gram negative micro-organisms other than the *Helicobacter* species to be cultured. More particularly, the at least one selective substance is selected from the group consisting of: vancomycin, trimethoprim lactate, polymyxin B, cefsulodin, colistin, amphotericin B, crystal violet, nystatin and nisin.

Further specific embodiments of the methods of the invention make use of cultivation systems comprising a solid and a liquid component, wherein the solid component comprises between 12.5 and 25 % serum or between 7.5 and 15%, more particularly between 10 and 15 % blood.

Further specific embodiments of the methods of the invention make use of cultivation systems comprising a solid and a liquid component as described above, wherein the solid and/or liquid component further comprises one or more growth factors selected from the group of Vitamin B12, L-glutamine, Adenine, Guanine, p-Aminobenzoic acid, L-cystine, NAD (Coenzyme 1), Cocarboxylase, Ferric nitrate, Thiamine and Cysteine hydrochloride.

The methods of the present invention are particularly suited for the isolation of *"Candidatus* Helicobacter suis". Indeed, the present invention provides, for the first time, a method for obtaining a *"Candidatus* Helicobacter suis" isolate, free from fungi or bacteria present in the stomach.

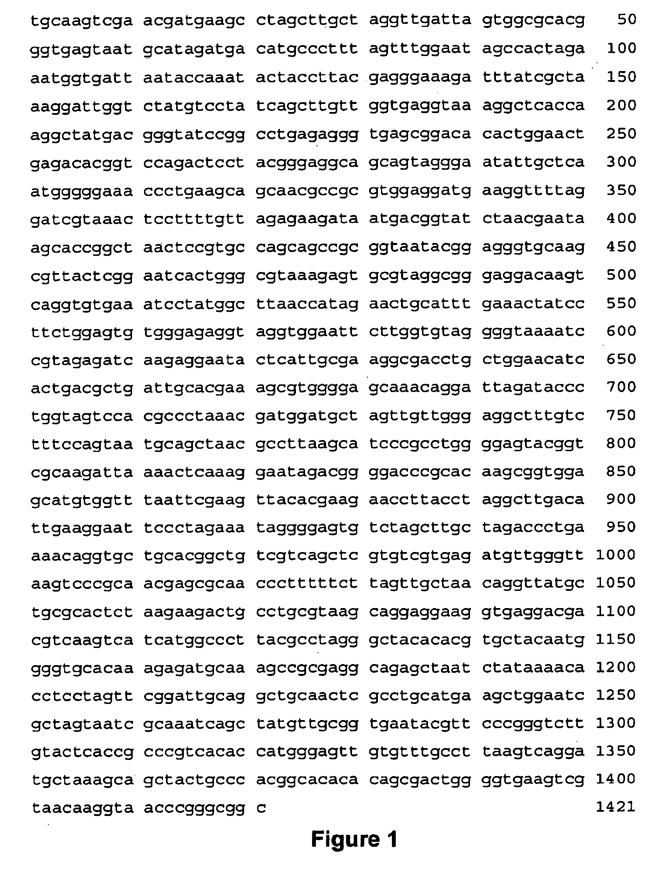

Accordingly, a second aspect of the present invention provides isolates of *"Candidatus* Helicobacter suis", , wherein the 16S rRNA gene sequence of *Helicobacter suis* has at least 99 % sequence identity with the 16S rRNA gene sequence of AF127028 Genbank accession number AF127028 (of November 1999) depicted in SEQ ID NO:7 and Figure 1, free from bacteria or fungi present in the stomach, obtainable by the above methods and which are free from fungi or bacteria present in the stomach. Such isolates, which are here demonstrated for the first time, are obtainable by the methods of the present invention.

A third aspect of the present invention provides methods for the cultivation of isolates of *Helicobacter* species, which methods comprise the step of applying the Helicobacter isolate in a cultivation system comprising a cultivation medium having a pH between 4.0 and 7.0 which is supplemented with at least 10 % serum or at least 7.5 % blood, and incubating the isolate therein.

In specific embodiments of the methods according to this aspect of the invention, the cultivation system used comprises a solid and a liquid component, and at least the solid component of this cultivation system comprises the above described cultivation medium.

In further specific embodiments of these methods of cultivation provided in the present invention, the solid and the liquid component comprise nutrients for the growth of fastidious bacteria.

In specific embodiments, at least the solid component of the cultivation system used in the methods of cultivation of the present invention, is buffered at a pH between 4.7 and 7.0.

Disclosed herein are cultivation recipients comprising a solid cultivation medium for the isolation and/or cultivation of *Helicobacter* species, the solid medium comprising: nutrients for the growth of fastidious organisms, blood in a concentration between 7.5 and 15 % or serum in a concentration between 12.5 and 25 %, and at least one selective substance that inhibits the growth of fungi.

Yet a further aspect of the present invention provides vaccines comprising an antigen preparation of a *"Candidatus* Helicobacter suis" isolate. Indeed, the present invention provides methods for producing antigen preparations of *"Candidatus* Helicobacter suis", which methods comprise the isolation and optionally cultivation of *"Candidatus* Helicobacter suis" according to the methods described above and the production of an antigen preparation from the obtained *"Candidatus* Helicobacter suis" isolate.

Additionally, the present invention provides vaccines comprising live attenuated *"Candidatus* Helicobacter suis", and/or whole killed *"Candidatus* Helicobacter suis" and methods for preparing such vaccines which comprise the isolation and optionally cultivation methods described above.

Herein disclosed are methods for vaccinating an animal against a *"Candidatus* Helicobacter suis" infection, which methods comprise administering to the animal a vaccine comprising one or more of an antigen preparation from a "*Candidatus* Helicobacter suis" isolate, live attenuated *"Candidatus* Helicobacter suis", and/or whole *"Candidatus* Helicobacter suis".

Similarly, the present invention envisages the use of a "*Candidatus* Helicobacter suis" isolate, which can be isolated and/or cultivated by the methods described herein, or the use of antigen preparations obtained from a *"Candidatus* Helicobacter suis" isolate, for the manufacture of a vaccine for the prevention and/or treatment of a *Helicobacter* species infection. More particularly, the use of a preparation of *"Candidatus* Helicobacter suis" is envisaged for the manufacture of a vaccine for the prevention and/or treatment of a *Helicobacter* species infection, most particularly for immunisation against "*Candidatus* Helicobacter suis".

### BRIEF DESCRIPTION OF THE FIGURES:

Figure 1 shows a partial sequence of Helicobacter suis 16S ribosomal RNA gene of Genbank Accession AF127028 as submitted on November 17, 1999. The same picture is also shown in SEQ ID NO 7.

### DETAILED DESCRIPTION OF THE INVENTION

The present invention will be described with reference to certain embodiments but the present invention is not limited thereto but only by the claims.

The terms *"Candidatus* Helicobacter suis" (or *Candidatus* H. suis or *H*. *suis*) as used herein refers to a bacterium that was previously known as *"Helicobacter heilmannii"* type I. (Trebesius et al. (2001) J. Clin. Microbiol. 39, 1510-1516). It is now accepted that "*H. heilmannii*" type 1 is identical to *"Candidatus* H. suis" (O'Rourke et al. (2004) Int. J. Syst. Evol. Microbio/. 54, 2203-2211; De Groote et al. (1999) Int. J. Syst. Bacteriol. 49, 1769-1777), a spirally shaped bacterium that colonises the stomach of more than 60% of slaughter pigs. *"Candidatus* Helicobacter suis" is also defined at the molecular level as the *Helicobacter* species having a 16S rRNA sequence Genbank Accession AF 127028 [SEQ ID NO:7 and Figure 1] (De Groote *et al.* (1999) cited above) and AF506788-92 (O'Rourke *et al.* (2004) cited above) and a urease gene sequence as depicted in Genbank Accession AF508013-AF508014 (O'Rourke et al. (2004) Int. J. Syst. Evol. Microbiol. 54, 2203-2211).

The term "isolate" as used herein, is a pure, homogeneous, in vitro culture derived from a heterogeneous, wild population of microorganisms.

The term "cultivation recipients" as used in the context of the present invention relates to a recipient suitable for cultivating micro organisms, such as but not limited to Petri dishes, culture flasks, roller bottles and cell factories. The term "antigen preparation" as used in the context of the present invention relates to a composition comprising at least one protein or fragment thereof which provokes an immune response (hereafter referred to as 'antigen') when administered to an animal.

The term "vaccine" as used herein refers to a composition such as an antigen preparation described above, for administration to an animal or human with the object of stimulating an immune response in the animal or human directed against a disease-causing organism, to protect the animal or human from illness or disease caused by that organism. Vaccines can comprise whole disease-causing organisms (killed or weakened) or parts of such organisms, or synthetic molecules corresponding to all or parts of such organisms. The term vaccine encompasses both compositions used for prophylactic use, i.e. for administration to the animal or human prior to infection, with the intent to prevent initial (and/or recurrent) infection and compositions for therapeutic use, i.e. for administration to the animal or human after infection with the intent to reduce or arrest disease progression caused by the organism.

The process of vaccinating whereby antigens from one species is used to protect against disease caused by another species using is referred to as "cross-vaccination" or "heterologous vaccination".

The term "gastric material" as used herein refers to any material obtained directly or indirectly from the gastrointestingal tract from human or other animals. Such materials include, for example, gastric epithelium, gastric mucosa, and digestive fluids.

The term "medium" as used herein refers to a liquid, solid or semi-solid composition suitable for the growth of microorganisms.

The term "fastidious organisms" is used herein according to its standard meaning in bacteriology, i.e. to refer to bacteria having complex nutritional requirements [Stedmans Medical Dictionary].

A first aspect of the invention provides *in vitro* methods for isolating and/or cultivating *Helicobacter* species, in particular *"Candidatus* Helicobacter suis". The *in vitro* isolation of *H. suis* has not yet been described to date. The present invention provides methods which allow the selective growth of *Helicobacter* species, more particularly the *"Candidatus* Helicobacter suis".

Selective growth conditions for *"Candidatus* Helicobacter suis" have been achieved by the combination of high concentrations of blood or serum and the provision of optimal pH conditions. The pH conditions which are beneficial for the growth of *Helicobacter* species are at the same time detrimental for a number of non-Helicobacter bacteria and fungi. By applying low pH, contamination is reduced, allowing *"Candidatus* Helicobacter suis" to expand.

In the isolation methods of the present invention, a sample comprising *Helicobacter* species is cultivated in a medium, the pH of which is adjusted to a value between about 5.0 and about 6.0. This pH adjustment can be performed by the addition of a concentrated acid to the medium nutrients, which provide sufficient buffering capacity, by the presence of amino acids which act as zwitter ions. Alternatively biocompatible buffers with a high buffering capacity around pH 5.0 to 5.5 (such as acetate, phosphate) are added to the cultivation medium. It was surprisingly found that the isolation of "*Candidatus* Helicobacter suis" at pH values between 5.0 and 6.0 resulted in spiral mobile bacteria, while outside this pH region, *Candidatus* H. suis appears as coccoid non-motile forms. As explained later in detail in the examples the effect of the pH on Candidatus H. suis changes to some extent with subsequent passaging. While the isolation of Candidatus H. suis is successful at a pH between 5.0 and 6.0.

According to one embodiment, the methods of the present invention comprise the application of a sample containing *Helicobacter,* more particularly *Candidatus* H. suis to a cultivation system, which is a two component cultivation system, comprising a solid component and a liquid (or semi-liquid) component, whereby the sample is provided in the liquid component. Typically the solid component comprises agar, and the liquid component is a broth comprising at least nutrients. It has been observed that in such cultivation systems, the Helicobacter isolate remains in the fluid fraction and can be subcultured by transferring to another solid component. Where a two-component system is used in the isolation methods of the present invention, at least the solid component has a pH between 5.0 and 6.0.

The cultivation medium in the isolation methods of the present invention comprises high concentrations of blood (between 7.5 and 25%) or a blood component such as serum (between 12.5 and 30%). Unless otherwise indicated serum and blood concentration are volume percentages (v/v) in the medium. In particular embodiments, the concentration of serum in the medium is between 12.5 and 25% including concentrations such as 12.5, 15, 17.5, 20 and 25%. In other particular embodiments, the concentration of blood in the medium is between 7.5 and 25%, more particularly between 8 and 20%, especially between 9 and 15%, including concentrations such as 9.5, 10 and 12.5%. Where a two-component cultivation system is used, at least the solid component, but optionally also the liquid component of the cultivation system should contain the specified serum or blood concentration.

The serum present in the cultivation medium used in the context of the present invention can be either foetal, new-born or adult serum of different species of animal, such as, but not limited to cattle, horse, sheep, goat, or pig, more particularly horse serum, or a combination thereof. Typically, serum contains *inter alia* iron-binding proteins such as albumin and transferrin, which provide iron to *Helicobacter.* Suitable serum or blood substitutes containing such essential components thereof can also be used.

Different types of blood are suitable for the methods of the present invention such as, but not limited to cattle, horse, sheep, goat or pig blood. In particular embodiments, the concentration of blood in the medium is between 7.5 and 15 % including concentrations such as 10 and 12.5 % blood.

According to particular embodiment, the cultivation media used in the initial isolation methods of the present invention further comprise antimicrobial agents further ensuring the selective growth of Helicobacter. Such antimicrobial agents include antibacterial agents and antifungal agents, which limit or inhibit growth of non-*Helicobacter* bacteria or yeast potentially present in the sample. Where a two-component system is used these anti-microbial agents are present at least in the solid component, and optionally also in the fluid or semi-fluid component.

In particular embodiments the cultivation medium used in the present invention comprises one or more antibiotics inhibiting the growth of fungi and/or Gram positive and/or Gram negative micro-organisms different from the *Helicobacter* species to be cultured. Examples of such antibiotics are vancomycin, trimethoprim lactate, polymyxin B, cefsulodin, colistin, amphotericin B, crystal violet, nystatin and nisin. A commercially available antibiotic composition which is suitable in the methods of the present invention is for example Skirrow supplement (Oxoid) resulting in a final concentration in the medium of 10 mg/ml vancomycin, 5 mg/l trimethoprim lactate and 2500 IU/I polymyxin B. Other antibiotic compounds are for example antifungal compounds such as Amphotericin B (fungizone).

Once an isolate of Helicobacter, such as *Candidatus* H. suis has been obtained, it can be further cultivated using the cultivation conditions described above for the isolation methods of the present invention. However, it has been observed that the cultivation of an isolated culture is possible at broader pH range, i.e. between 4.0 and 7.0. The pH is more critical in the isolation procedure as, at a pH above 6.0, the contaminants present in freshly isolated stomach samples (other bacteria and fungi) will outgrow the Helicobacter present in the sample, which is detrimental to isolation. It has been observed however, that for isolates of Candidatus H. suis containing less contaminants, growth of a pH up to 7.0 is possible. Accordingly, the present invention further provides cultivation methods for isolates of Helicobacter, more particularly *Candidatus* H. suis, as described above, wherein the pH of the cultivation medium is between about 4.0 and about 7.0, in particular between (and including) about 4.7 and 6.0.

The antibiotics and antifungals described above, are especially suitable in the isolation process wherein different bacteria and fungi are present in a sample. However, once a pure isolate is obtained the cultivation can be achieved with less or without antibiotics or antifungals in the cultivation medium.

In particular embodiments of both the isolation and/or cultivation methods of the present invention, the cultivation medium further comprises additional growth factors. A commercial mixture of growth factors is for example sold under the name of Vitox (Oxoid). Final concentrations of growth factors of this commercial composition in a growth medium (agar or broth) are vitamin B12 (0.5 mg/l), L-glutamine (50.0 mg/l), adenine (5.0 mg/l), guanine (0.15 mg/l), p-aminobenzoic acid (0.65 mg/l), L-cystine (5.5 mg/l), NAD Coenzyme-1 (1.25 mg/l), cocarboxylase (0.5 mg/l), ferric nitrate (0.1 mg/l), thiamine (0.015 mg/l) and Cysteine hydrochloride (130.0 mg/l). Where a two-component system is used, the growth factors can be present in either the solid component or the liquid component or both. According to a particular embodiment, the growth factors are added to the solid component.

Typically, the medium used in the isolation and/or cultivation methods of the present invention contains nutrients for the growth of fastidious organisms. These nutrients are typically provided by the addition of media such as, but not limited to, Brucella medium, Mueller-Hinton medium, bovine and porcine Brain Heart Infusion and equivalent media, such as, but not limited to Columbia agar, Tryptic Soy agar. Brucella media typically comprise in a litre of medium 10 g pancreatic digest of casein, 10 g peptic digest of animal tissue, 1 g dextrose, 2 g yeast extract, 5 g sodium chloride and 0.1 g sodium bisulfite. Herein peptones supply organic nitrogen. The yeast extract is a potent source of the B vitamins. Dextrose is utilised as an energy source. Mueller-Hinton medium typically comprises in a litre of medium 2 g meat infusion, 17.5 g casein hydrolysate and 1.5 g starch. Such media are commercially available from companies such as Difco and Oxoid.

The present invention provides methods for the isolation and/or cultivation of *Helicobacter* species, which allow for the isolation and cultivation of a true Helicobacter isolate, more particularly a *"Candidatus* Helicobacter suis" isolate. It is understood, however, that other factors exist which influence the enrichment of *Helicobacter* species Such factors include the way the sample comprising the *Helicobacter* species is obtained, more particularly, the way in which the stomach and the stomach wall is treated upon retrieval of the gastric material to minimise the contamination with other organisms.

Accordingly, particular embodiments of the isolation methods of the present invention further comprise, a first step of providing a sample of gastric material comprising *Helicobacter* species, under specific conditions. In one embodiment, a part of the stomach wall is incubated in acidic conditions which kill a considerable number of acid-labile organisms. In a further particular embodiment the stomach wall is processed by isolating only the surface mucus of the stomach.

The media used in the cultivation and/or isolation methods of the present invention, optionally comprise one or more additional components.

In particular embodiments of the methods of the present invention the cultivation medium further comprises active charcoal. Where a two-component system is used, the active charcoal is typically present at least in the solid component.

In particular embodiments of the methods of the present invention, the cultivation medium further comprises a biocompatible pH indicator which colour changes at a pH between 5.5 and 6.0. For instance, phenol red which turns yellow below pH 6.6 and red above pH 8 could be used. Such indicators are especially useful in plates comprising serum. Typically, in a two-component system, such a pH indicator is present in the solid component.

In particular embodiments, the methods of the isolation and/or cultivation methods of the present invention comprise the application of a sample comprising *Helicobacter* species on a two component system, wherein the solid component comprises nutrients, growth factors and antibiotics, and is adjusted to a pH value between 4.7 and 7.0 (or more particularly between 5.0 and 6.0), while the liquid component only comprises nutrients without adjustment of the pH. Optionally the pH of the liquid component is adjusted to a pH value between 4.7 and 7.0 (or, more particularly, between 5.0 and 6.0) and/or the liquid component also comprises growth factors and antibiotics.

Particular practical configurations can be considered for carrying out the methods of the present invention. Typically, isolation and cultivation is carried out in cultivation flasks or petri-dishes (plates). In particular embodiments the methods involve a two component system, whereby the solid component covers the entire bottom of the cultivation recipient (with a typical thickness of between 1 mm and 5mm) and the liquid or semi-liquid component is spread out over all or a part of the surface of the solid component.

In a very particular embodiment, the isolation is achieved in a petri-dish-like recipient with a diameter of 10 cm comprising between about 5 to 15 ml of a solid agar with the composition as described above. On top of the agar between about 500 to 1000 µl of liquid broth is added as liquid component.

In another particular embodiment, the two-component system which is used comprises a bottom agar without added nutrients, serum, or other additives, but which comprises a buffer at pH 5. On top of this bottom agar, a top agar is applied which contains all necessary ingredients for the cultivation of *"Candidatus* Helicobacter suis". Using this buffer a minimum of expensive ingredients is used, while the pH of the top agar is kept stable by the presence of the underlying bottom agar.

The methods of the present invention can be used for the isolation and/or cultivation of *Helicobacter* species, more particularly *"Candidatus* Helicobacter suis". Typically, in the methods of the present invention, the sample of gastric material is incubated in the cultivation system for a period between 1 to 15 days, depending on the degree of contaminating micro-organisms and the nutrient utilisation of the medium. Where a solid medium is used, a sign of exhaustion of the medium is typically the appearance of translucent areas in the solid medium. Additionally or alternatively, the motility of *Helicobacter* species in the liquid medium can be assessed, whereby a reduced motility is indicative of exhaustion of the medium. Additionally or alternatively, the bacterial isolate can be transferred to a fresh plate when the pH of the cultivation medium (or the solid and/or liquid component) increases to a pH value of 6.0 or more.

Typically, the methods of the present invention involve the incubation of samples comprising *Helicobacter* species or isolates of *Helicobacter* species at 37 °C. It was found that below 25 or above 40° degrees no growth of *Helicobacter* bacteria occurs. Generally plates with *Helicobacter* species are grown under micro-aerobic conditions, i.e. between 2 and 8 % oxygen, more particularly about 4-5% O₂. However, *Helicobacter* species do not grow under complete anaerobic conditions.

A particular embodiment of the methods for the isolation of *Helicobacter* species from a sample comprises the steps of:
a) applying the sample in a cultivation system comprising a solid and a liquid component,
   - wherein at least the solid component is buffered at a pH between 5.0 and 6.0, and
   - wherein the solid component comprises at least 10 % serum or at least 7.5
      % blood, and
   - wherein the solid and the liquid component comprise nutrients for the growth of fastidious bacteria, and
   - wherein the solid and/or liquid component comprise at least one selective substance that inhibits the growth of fungi and/or Gram positive and/or Gram negative micro-organisms different from the *Helicobacter* species to be cultured,
      the method further comprising the step of
b) incubating the sample in this system under micro aerobic conditions.

Using the above described methods for the isolation and cultivation of *Helicobacter* species, true isolates of *Helicobacter* species, in particular *"Candidatus* Helicobacter suis", can be obtained from samples of gastric material. In addition, the methods of the present invention allow cultivation *in vitro* in bulk. It has been demonstrated that using the methods of the present invention, about 1 mL stomach mucus applied on one 10 cm bacterial plate generates a culture of between about 10⁶ to 10⁹ bacteria/ml within 3 days. Upon passaging cultures can be diluted with a factor of 1 to 5. Accordingly, even with small-scale systems, high yields can be reached. However, in large-scale production, the yields can be further increased.

The methods of the present invention for the first time allow the generation of large amounts of true *Helicobacter* species isolates, in particular of *"Candidatus* Helicobacter suis". This is important as it allows the manufacture of vaccines using isolated *"Candidatus* Helicobacter suis" or antigen preparations obtained therefrom.

Accordingly, another aspect of the invention relates to the use of *"Candidatus* Helicobacter suis", such as those obtainable by the methods described herein for the production of antigenic preparations, which are useful as vaccines. Indeed, the present invention allows, for the first time, the generation of a vaccine based on true *"Candidatus* Helicobacter suis" isolates.

Antigen preparations of *"Candidatus* Helicobacter suis" isolates envisaged in the context of the present invention include both whole-cell bacterial preparations as preparations of components of *"Candidatus* Helicobacter suis" isolates. Such antigen preparation may comprise whole-killed (inactive) bacteria, live-attenuated (weakened) bacteria or processed and/or artificial bacterial preparations or combinations thereof. Processed bacterial preparations include preparations of bacterial proteins, which are partially or completely purified and/or pre-treated. These can be used alone or in combination with artificial antigen preparations such as protein preparations which are either in part or entirely obtained by synthetic or recombinant methods.

According to a particular embodiment, the antigen preparations provided by the present invention comprise one or more antigens obtained from a *"Candidatus* Helicobacter suis" isolate. The advantage of working with antigen preparations obtained from isolates is the purity, which reduces the chance of contamination with antigenic compounds of other organisms or bacterial species, thereby increasing the risk of unwanted side-effects of the antigenic preparation when injected into humans or other animals.

According to a particular embodiment, the antigen preparation is a cell lysate of *"Candidatus* Helicobacter suis", i.e. a mixture obtained upon lysis of bacterial cells. A particular example of a bacterial cell lysate is the soluble fraction of a sonicated bacterial culture, e.g. obtained after filtration. Alternatively or in addition, bacteria can be fragmented using a high-pressure homogeniser (e.g. Avestin model EmulsiFlexC5).

Optionally, the cell lysate is further inactivated prior to or after sonication by one of a variety of known methods, such as but not limited to, treatment with formalin, binary ethyleneimine (BEI), beta propriolactone (BPL), gluteraldehyde, irradiation or heat. Generally not all proteins in a lysate will provoke an immune response. Alternatively, the antigen preparation according to the present invention is obtained by fractionation and/or purification of one or more proteins from a lysate or bacterial culture medium to obtain a composition of enriched or purified antigens. Examples of isolated or purified bacterial proteins suitable in the context of the present invention are heat shock proteins and/or urease proteins, cagA and vacA.

Accordingly, another aspect of the present invention provides vaccines comprising the antigen preparations obtained from *"Candidatus* Helicobacter suis" isolates as described above. The vaccines of the present invention comprising an antigen preparation of a *"Candidatus* Helicobacter suis" isolate, can be used to obtain prophylactic or therapeutic immunity to infection by a *Helicobacter* species, more particularly by "*Candidatus* Helicobacter suis".

The vaccine of the present invention optionally contains only the antigen preparation of the invention. Alternatively, the vaccine can comprise, in addition to the antigen preparation of the present invention, a suitable adjuvant. For example, the antigen preparations in the vaccines of the present invention can be formulated in or with liposomes, preferably neutral or anionic liposomes, microspheres, ISCOMs, or virus-like particles (VLPs), in order to promote the screening of the protein or of the polypeptide or to increase the immune response. Persons skilled in the art have these compounds available without difficulty; for example see Liposomes: A Practical Approach. RRC New Ed, IRL press (1990).

Adjuvants other than liposomes may also be used. A large number are known to persons skilled in the art. The type of adjuvant will vary, depending on the type of antigen preparation and route of administration used. According to a particular embodiment of the present invention the antigen preparation is a sonicated antigen solution which is administered intranasally with Cholera toxin (CT) or subcutaneously with saponin as adjuvant. Any adjuvant known in the art may be used in the vaccine composition, including oil-based adjuvants such as Freund's Complete Adjuvant and Freund's Incomplete Adjuvant, mycolate-based adjuvants (*e.g*., trehalose dimycolate), bacterial lipopolysaccharide (LPS), peptidoglycans (*i.e*., mureins, mucopeptides, or glycoproteins such as N-Opaca, muramyl dipeptide [MDP], or MDP analogs), proteoglycans (*e.g*., extracted from *Klebsiella pneumoniae*), streptococcal preparations (*e.g.,* OK432), Biostim™ (*e.g*., 01K2), the "Iscoms" of EP 109 942, EP 180 564 and EP 231 039, aluminium hydroxide, saponin, DEAE-dextran, neutral oils (such as miglyol), vegetable oils (such as arachid oil), liposomes, Pluronic® polyols. Adjuvants include, but are not limited to, the RIBI adjuvant system (Ribi Inc.), alum, aluminium hydroxide gel, cholesterol, oil-in water emulsions, water-in-oil emulsions such as, e.g., Freund's complete and incomplete adjuvants, Block co-polymer (CytRx, Atlanta GA), SAF-M (Chiron, Emeryville CA), AMPHIGEN® adjuvant, saponin, Quil A, QS-21 (Cambridge Biotech Inc., Cambridge MA), GPI-0100 (Galenica Pharmaceuticals, Inc., Birmingham, AL) or other saponin fractions, monophosphoryl lipid A, Avridine lipid-amine adjuvant, heat-labile enterotoxin from *E*. *coli* (recombinant or otherwise), cholera toxin, or muramyl dipeptide, among many others. According to a particular embodiment recombinant mutant of *Escherichia coli* heat-labile toxin is added to the antigen preparation prior to injection into the animal.

For parenteral administration, specific components may be added such as aluminum hydroxide, aluminum phosphate and aluminum hydroxyphosphate. One or more antigens of the antigen preparation may be absorbed or precipitated on an aluminum compound according to standard methods. Other adjuvants useful for parenteral administration include in particular polyphosphazene (WO 95/2415), DC-chol (3-beta-[N-(N', N'-dimethylaminomethane) carbamoyl) cholesterol] (U.S. Pat. No. 5,283,185 and WO 96/14831), QS-21 (WO 88/9336) and RIBI.

The components of the vaccines of the present invention may be manufactured conventionally. In particular, a polypeptide, a mixture or a molecule of DNA contained in the composition according to the invention is combined with a pharmaceutically acceptable diluent or carrier, e.g. water or a saline solution such as phosphate-buffered saline (PBS). In general, the diluent or the carrier is selected on the basis of the mode and route of administration and of standard pharmaceutical practices. Pharmaceutically acceptable diluents and carriers as well as all that is necessary for their use in pharmaceutical formulations are described in Remington's Pharmaceutical Sciences.

The vaccines of the present invention can be in solid or liquid form such as, tablets, capsules, powders, solutions, suspensions, or emulsions.

The solid unit dosage forms can be of the conventional type. The solid form can be a capsule, such as an ordinary gelatin type containing the proteins or peptides of the present invention and a carrier, for example, lubricants and inert fillers such as, lactose, sucrose, or cornstarch. In another embodiment, these compounds are tableted with conventional tablet bases such as lactose, sucrose, or corn starch in combination with binders like acacia, corn starch, or gelatin, disintegrating agents such as, corn starch, potato starch, or alginic acid, and a lubricant like stearic acid or magnesium stearate.

The vaccines of the present invention may also be administered in injectable dosages by solution or suspension of these materials in a physiologically acceptable diluent with a pharmaceutical carrier. Such carriers include sterile liquids such as water and oils, with or without the addition of a surfactant and other pharmaceutically acceptable adjuvants. Illustrative oils are those of petroleum, animal, vegetable, or synthetic origin, for example, peanut oil, soybean oil, or mineral oil. In general, water, saline, aqueous dextrose and related sugar solution, and glycols such as, propylene glycol or polyethylene glycol, are preferred liquid carriers, particularly for injectable solutions.

For use as aerosols, the vaccines of the present invention comprising the antigen preparation in solution or suspension may be packaged in a pressurised aerosol container together with suitable propellants, for example, hydrocarbon propellants like propane, butane, or isobutane with conventional adjuvants. The materials of the present invention also may be administered in a non-pressurised form such as in a nebuliser or atomiser.

The vaccine comprising an antigen preparation of *"Candidatus* Helicobacter suis" can be used for autologous or heterologous vaccination. In autologous vaccination using the vaccines of the present invention, the object is the protection against infection by "*Candidatus* Helicobacter suis". In heterologous vaccination, the object is to obtain, by injection with an antigenic preparation of "*Candidatus* Helicobacter suis", protection against infection by one or more other *Helicobacter* species.

Accordingly, a further aspect of the present invention provides methods for the vaccination of humans and other animals using the vaccines of the present invention. The object of the vaccination schemes with the vaccines obtained from "*Candidatus* Helicobacter suis" isolates according to the present invention includes obtaining complete protection (sterilising immunity) against *Helicobacter spp*., more particularly against "*Candidatus* Helicobacter suis" in an animal but also reducing the bacterial burden of *Helicobacter spp.,* more particularly of "*Candidatus* Helicobacter suis" by at least 25, 40, 60, 80% compared to prior to vaccination and/or compared to animals which have not received the vaccine of the present invention and are/have been subjected to the same infectious agent. Most particularly, the present invention relates to vaccines and vaccination strategies which ensure a protective effect or reduced bacterial burden for a prolonged period of time, such as during at least 4, 6, 10, 12 or more than 12 weeks.

The administration of the vaccines of the present invention may take place in a single dose or in a dose repeated once or several times after a certain period. The appropriate dosage varies according to various parameters, for example the individual treated (adult or child), the vaccinal antigen itself, the mode and frequency of administration, the presence or absence of adjuvant and, if present, the type of adjuvant and the desired effect (e.g. protection or treatment), as will be determined by persons skilled in the art. For example, where antigen preparations comprising inactivated whole *"Candidatus* Helicobacter suis" are used, suitable dosages include, but are not limited to dosages of 10µg, 50µg, 100 µg, 250 µg, 500µg and 1 mg antigen preparation. Alternatively, dosages can be expressed in CFUs, 100µg corresponding to about 10⁷ CFU. Crude antigen preparations (i.e. containing traces of culture medium etc.) may require higher dosages to be effective than partly purified or purified preparations.

The methods of vaccination may include administration to the animal or human of one or more compositions prior to or after (oral) administration of the vaccine comprising the "*Candidatus* Helicobacter suis" antigen preparation according to the invention. Such components include, compounds which reduce acid production in the stomach (e.g. Cimetidine (Tagamet®, GlaxoSmithKline; Genval, Belgium)).

The identification and quantification of infection and/or bacterial burden in an animal can be done in a number of ways. Classically, this is done by determining the presence of the infectious agent, or a protein or DNA sequence thereof in a sample of body fluid or in urine or faeces. Alternatively, the reaction of the immune system, e.g. the presence of antibodies to the infectious agent, can be measured. An accurate diagnosis and quantification of *Helicobacter* infection is obtained by identification of "*Candidatus* Helicobacter suis" DNA, e.g. by PCR as described in the art (Fox and Lee (1997) Lab. Anim. Sci. 47, 222-255). Alternatively a quantitative urease test is uses to quantify *Candidatus* H. suis. Briefly mucosal tissue samples (approximately 0.5cm²) are immersed in 1,000 µl of CUTest (Temmler Pharma; Marburg, Germany) and incubated at 37°C for approximately 3 hours (as described by Corthésy-Theulaz et al., (1995), Gastroenterology 109, 115-121). After centrifugation, the supernatant is used for spectrophotometric quantification at an optical density (OD) of 550 nm. The cut-off for discrimination between infection or not is calculated for each stomach region and corresponded to the mean plus two times the standard deviation (SD) of the absorbance values obtained with gastric biopsy specimens from non-challenged control animals. Values above this cut-off are considered evidence of colonization by *"Candidatus* Helicobacter suis".

As detailed above, the use of the vaccines of the present invention is envisaged both in autologous and heterologous vaccination schemes. Methods are provided for the treatment and/or protection of humans and/or other animals against infection by one or more different *Helicobacter* species, optionally including *"Candidatus* Helicobacter suis". Particular embodiments of the present invention relate to the use of the vaccines obtained from of "*Candidatus* Helicobacter suis" isolates to obtain prophylactic or therapeutic immunity to other *Helicobacter spp.* such as, but not limited to, *H. pyloris, H. bizzozeronii, H. felis* and H. *salomonii.* Other suitable *Helicobacter* species *are H.bilis, H. fenelliae, H. pametensis, H. nemestrinae, H. nemestrinae, H. pametensis, H. acinonychis, H. pullorum, H. mustelae, H. hepaticus, H. cinaedi* and *H. canis.*

The invention provides methods of prophylactic and/or therapeutic protection against infection with *"Candidatus* Helicobacter suis", which comprise administering the vaccines of the present invention obtained from *"Candidatus* Helicobacter suis" isolates. More specifically, antigen preparations are provided for use in prophylactic vaccination, which ensure protection against *Helicobacter* spp., more particularly against *"Candidatus* Helicobacter suis", which protection is more than transient.

Also provided are methods for therapeutic immunisation, when the organisms have already orientated the host immune response to their benefit.

The methods of immunization include methods whereby the vaccine is administered through any suitable route, such as by mucosal (intranasal), parenteral, or intramuscular administration, oral, intradermal, intraperitoneal, intravenous, transdermal/transcutaneous, or subcutaneous administration. Suitable vaccination routes also comprise combination administrations (e.g. oral/ intramuscular administration). Therapeutic immunisation can be performed by parenteral administration of the antigen preparations or vaccines of the invention. Parenteral immunisation can mobilise cells from systemic origin that have not been already primed in one given direction by a *Helicobacter* infection (Guy et al. (1999) Vaccine 17, 1130-1135).Intramuscular administration can be used for efficient vaccination.

The different aspects of the present invention are illustrated by the examples detailed hereafter.

### EXAMPLES

### Example 1: In vitro isolation and cultivation of "Candidatus Helicobacter suis"

In this example, *"Candidatus* Helicobacter suis" is isolated using a two component cultivation system, whereby the solid component is a Brucella agar plate.

Brucella agar plates (Becton-Dickinson, Erembodegem, Belgium) were supplemented with Vitox supplement (Oxoid, Basingstoke, UK), Skirrow supplement (Oxoid), 20% foetal calf serum (FCS; QB Perbio Tattenhall, UK), 0.1% activated charcoal GR (AC; Merck, Darmstadt, Germany), 0.00001% crystal violet (Clin-Tech Ltd, Essex, UK) and 0.001% Nisin (Sigma-Aldrich, St-Louis, Mo, USA). After autoclaving but before gelling of the agar, 0, 0.2, 0.5 and 0.7 ml of a HCl solution (min. 37%; Riedel-de Haën, Seelze, Germany) was added to 500 ml of Brucella agar, resulting in pH values of 7.0, 6.3, 5.5 and 5.0, respectively. Unless otherwise indicated Brucella plates with serum comprise 20% serum.

In this example the liquid component of the two component cultivation system is Brucella broth, without addition of other supplements, serum and without adjustment of pH.

### Day 0

Five swine stomachs (A to E) were collected from the slaughterhouse (Porc Meat N.V., Zele, Belgium) and stored at +4°C until further use. They were opened at the *curvatura major* and rinsed with tap water. One half of the stomach was subjected to acid treatment (submersion in a 1% HCl bath for 1 hour). Thereafter, only the surface mucus was collected by scraping the stomach with a glass slide. This mucus was collected into a sterile tube. Microscopic examination of the stomach mucus of the pigs, showed that all stomachs were positive for *"Candidatus* Helicobacter suis".

The mucus was slightly liquefied with the Brucella broth comprising 20 % serum (up to a volume of about 5 mL) and inoculated on four supplemented Brucella agar plates with serum with pH values of 5.0, 5.5, 6.3 and 7. The sample was inoculated onto the middle of the plate, and subsequently three drops of Brucella broth ( about 150 µl) were added onto the middle of the mucus sample.

Four plates per pig in total were incubated overnight in a micro-aerobic atmosphere at 37°C. The micro-aerobic environment was created by evacuating 80% of the normal atmosphere and introducing a gas mixture of 8% CO₂, 8% H₂ and 84% N₂.

### Day 1

Primary isolation of motile spiral bacteria (indicating the presence of *Helicobacter* species) was successful for four of the five stomach samples. For stomach D, there was insufficient growth on day 1. Plates which were dried out, were slightly humidified using Brucella broth with serum.

### Day 2

It was observed that for all stomachs, the plates in which the agar was at pH 7 and 6.3 were contaminated with other bacteria. Broth from these plates was filtered (using a 0.65 µm pore filter) and passaged onto new agar plates of the same pH.

Only some of the plates with pH 5.5 or 5.0 were contaminated, in which case the broth was also filtered and passaged to new plates. When no contamination was seen, the plates were further incubated. This was the case for the plates at pH 5.0 of stomach samples D and E.

When the pore size of the 0.65 µm pore filters was too small to filter the medium without clogging, the filtration was performed with 0.8 µm filters. This was the case for contaminated plates of pH 7, 6.3 and 5.5 of stomach sample E.

### Day 3

Those plates which were inoculated with filtered medium on day 2, were mostly negative for contaminating bacterial growth.

The original non-contaminated, non-filtered pH 5.0 plate from stomach E was analysed microscopically and showed many "*Candidatus* Helicobacter suis" bacteria with a remarkable motility, the number of which was estimated to be about 10⁸ or 10⁹/ml. The broth culture (about 500 µl) was transferred onto a new agar medium of pH 5.

### Day 4

The original pH 5.0 plate of stomach E and its passaged plate, were overgrown with contaminants. Broth from these plates was transferred onto new agar plates using 0.65 µm pore filters.

Many plates from the other stomachs were also contaminated and discarded. From stomach A, two filter-passaged plates were negative for bacterial contamination and were further incubated. From stomachs B, C and E, one filter-passaged plate was negative and further incubated.

From stomach D, the pH 5.0 plate was still negative for contaminating bacterial growth and was further incubated.

### Day 5

All filter-passaged plates, which were negative on day 4 for *"Candidatus* Helicobacter suis", were still negative for *Helicobacter* and were further incubated. Some plates showed contaminating bacteria.

### Day 7

The broth on top of the pH 5.0 plate from stomach D showed viable and motile *"Candidatus* Helicobacter suis" bacteria, the number of which was estimated to be 10⁶/ml. The mucus was not visibly contaminated with other bacteria. This plate was further incubated.

### Day 8

From stomach D, the original plate of pH 5.0 showed further growth of *"Candidatus* Helicobacter suis" bacteria. The broth was transferred onto an agar plate of pH 7. On the original plate, 500 µl of Brucella broth with serum was added.

### Day 11

The passage of sample D of day 8 onto a pH 7 plate was negative for *Helicobacter* growth. The original plate of pH 5.0 still showed a viable culture of *"Candidatus* Helicobacter suis" (estimated 10⁸ bacteria/ml). This broth was transferred onto a pH 5.0 plate, and an equal amount of Brucella broth with serum was added.

On the original plate, 500 µl of Brucella broth with serum was added.

### Day 13

The passage on the pH 5.0 plate on day 11 was successful for the isolate originating from sample D. The broth contained motile *Helicobacter* (estimated 10⁸ bacteria/ml). Broth was again transferred onto a pH 5.0 plate, while the plate of the first passage was humidified with 1 ml of Brucella broth with serum. The broth on the original plate of sample D also contained a viable culture, of which about 200 µl was frozen in an equal amount of LYM (¼ BHI broth +¾ horse serum + 7.5 % (w/v) glucose) at -70°C. Again 500 µl of Brucella broth with serum was added before further incubation.

From stomachs B and C, filter passages on day 4 onto plates of pH 5, were slightly positive for *"Candidatus* Helicobacter suis".

### Day 14

The frozen culture D of day 13 was thawed and applied on a supplemented Brucella agar plate with pH 5, and incubated in a micro-aerobic atmosphere.

### Day 15

The second passage of sample D (HS1) was positive for *"Candidatus* Helicobacter suis" (estimated 10⁸ bacteria/ml). Broth of this plate (about 500 µl) as well as broth from the original sample D pH 5.0 plate was added to an equal volume of Brucella broth, 3 vol. FCS and 7.5 % (w/v) glucose, and frozen at - 70°C.

The first passage contained more broth (about 1 ml) and was passaged onto two fresh agar plates of pH 5. Equal amounts of Brucella broth with serum were added.

The filter passage of B was still slightly positive for *Helicobacter,* whilst the filter passage of C contained about 10⁶ bacteria/ml. These plates were slightly humidified with Brucella broth with serum.

### Day 17

The original pH 5.0 plate of sample D was again positive for *Helicobacter,* but they were less motile. As the medium of the agar was probably exhausted, the broth was transferred again onto a fresh agar plate (pH 5) with medium and the original plate was discarded.

The second passage of D was positive and broth was again transferred onto a new agar plate, and the plate was again humidified with supplemented Brucella broth and further incubated.

Of the filter-passaged plates of B and C, broth was transferred onto new agar plates of pH 5. On the original plates Brucella broth with serum was added.

### Day 19

Samples were taken from cultures derived from stomachs D (isolate HS1), C (isolate HS2) and B (isolate HS3). DNA was extracted from bacterial cells using DNeasy Tissue kit (Qiagen). PCR was performed using *Candidatus* H. suis-specific primers (De Groote et al., 1999 Bacteriology 49, 1769-1777; 2000 J. Clin. Microbiol. 38, 1131-1135). Agarose gel electrophoresis showed a 433 bp long PCR fragment for all three isolates. DNA from a culture of *in vivo* mouse passaged *"Candidatus* Helicobacter suis" was used as a positive control.

The plate inoculated on day 14 with thawed material from HS1, was positive for *"Candidatus* Helicobacter suis". The broth was transferred on two new plates (second passage).

The second passage of HS1 was mixed with an equal amount of 1 vol. Brucella broth, 3 vol. FCS, 7.5 % (w/v) glucose and frozen at -70°C.

The broth from the third passage of HS1 was positive and transferred onto a new plate.

### Day 20

For HS2 and HS3, the broth from the second passages was mixed with an equal amount of 1 vol. Brucella broth, 3 vol. FCS, and 7.5 % (w/v) glucose and frozen at -70°C.

### Day 22

On the plate inoculated with thawed material from HS1, *"Candidatus* Helicobacter suis" was not grown much further. The passages of day 19 of this isolate however, showed more *Helicobacter.*

The fourth passage of day 19 from the original culture did not show many bacteria.

The second passages of day 17 of cultures deriving from C and D showed many *"Candidatus* Helicobacter suis" bacteria. The broth from sample C (HS2) was transferred onto two new plates, and equal volumes of supplemented Brucella broth were added. The broth from sample B (HS3) was transferred onto one new plate, and an equal volume of supplemented Brucella broth was added.

### Day 25

For HS1, the second passage of day 15 showed very few *Candidatus* H. suis. All broth was transferred to a fresh agar plate (pH 5.0) and the old plate was discarded.

The third passage of day 17 and the fourth passage of day 19 were negative and discarded.

The plate inoculated on day 14 with thawed material from HS1, showed a grown culture of *"Candidatus* Helicobacter suis" and this broth was inoculated onto two new plates and with Brucella broth with serum. The second passages of day 19 of this isolate, were also inoculated onto two plates each.

For HS2 and HS3, the second passages of day 17 showed less motile *Helicobacter.* The plates showed translucent areas and were probably exhausted. All broth was transferred onto a new plate and the old plate was discarded. The third passages showed good cultures which were transferred onto two new plates per isolate.

### Day 27 and following days

From the third and fourth passages of HS1, broth was frozen in an equal volume of 1 vol. Brucella broth, 3 vol. of FCS and 7.5 % (w/v) glucose. The broth of the second passage of day 22 was inoculated onto a new agar plate.

For HS2 and HS3, third and fourth passages were inoculated onto a new agar plate. Cultures were passaged continuously every two or three days and broth cultures were frozen regularly as described above.

16S rRNA gene of isolates HS1, HS2 and HS3 was amplified using primers complementary to the conserved edges. Consensus primers αβ-NOT (5'-tcaaactaggaccgagtc-3') [SEQ ID NO:1] and ωMB (5'-taccttgttacttcacccca-3') [SEQ ID NO:2] were used, as previously described (Baele et al., (2001) J. Appl. Microbiol. 91, 488-491). A 1500 bp amplicon amplified in this PCR reaction was sequenced using primers pD (5'-cagcagccgcggtaatac-3') [SEQ ID NO: 3], γ* (5'-ctcctacgggaggcagcagt-3') [SEQ ID NO:4], 3 (5'-gttgcgctcgttgcgggact-3') [SEQ ID NO: 5] and O* (5'-aactcaaaggaattgacgg-3') [SEQ ID NO: 6], as described in Coenye et al. (1999) Int. J. Syst. Evol. Microbiol. 49, 405-413). Sequence analysis was performed using the ABI Prism^{™} 3100 Genetic Analyzer (Applied Biosystems, Lennik, Belgium) and sequences were aligned with GenBank using BLAST. The sequence revealed 99% similarity with the sequence of *"Candidatus* Helicobacter suis" in GenBank (AF127028 depicted in SEQ ID NO:7 and Figure 1).

In the experimental settings as described above, primary isolation was most successful on plates containing medium of lower pH values (5.0 or 5.5). Under these conditions the growth of contaminating bacteria was diminished.

Isolation of *"Candidatus* Helicobacter suis" from stomach E was successful. On the third day after inoculation, a pure culture of about 10⁸ or 10⁹/ml was seen under the microscope. Contaminants growing on the mucus were mixed with the broth containing *Helicobacter,* which was unavoidable, when transferring the broth onto a new agar plate.

From stomachs B, C and D, pure cultures of *"Candidatus* Helicobacter suis" were obtained. A species-specific PCR amplifying a 16S rDNA fragment confirmed the species identity. Sequencing of the whole 16S rDNA gene revealed 99% similarity with the sequence of *"Candidatus* Helicobacter suis" in GenBank.

For isolate HS1, originating from D, primary isolation of a pure broth culture of "*Candidatus* Helicobacter suis" was obtained after 7 days of incubation. Transferring the broth onto a plate with the same medium at pH 7 did not result in growth. Passaging to a plate of pH 5.0 was successful. Broth showing viable and motile *Helicobacter* was divided into two equal volumes and transferred onto two fresh agar plates. Supplementation with an equal amount of Brucella broth (with 20% FCS) resulted in good growth of the bacteria.

"*Candidatus* Helicobacter suis" cells in broth can be frozen at -70°C in an equal amount of 1 vol. Brucella broth, 3 vol. FCS and7.5 % (w/v) glucose. After thawing one vial that was frozen, *Candidatus* H. suis was successfully regrown on the same medium.

For isolates HS2 and HS3, originating from stomach B and C respectively, primary cultures on media with pH 5.0 were slightly contaminated with other bacteria. The broth was then brought onto a 0.65 µm pore filter and inoculated on new agar plates of pH 5. After 11 days of incubation, these plates were positive for *Helicobacter* growth. After again 4 days of incubation, the broth contained enough *Helicobacter* to passage the strains. Second and third passage cultures were obtained after 5 and 3 days of incubation respectively.

### Example 2: Effect of temperature and environment on "Candidatus Helicobacter suis" growth.

In this and all following experiments, the optimisation of culture medium and growth conditions was evaluated on "*Candidatus* Helicobacter suis" isolates HS1, HS2 and HS3. Details on the isolation of these strains are given in example 1.

Isolates HS1 and HS3 were inoculated each onto six Brucella agar plates as described in the previous example, (i.e. comprising 20% FCS and adjusted to pH 5.0). Plates were overlaid with Brucella broth supplemented with Vitox supplement, fungizone, 20% FCS and 0.7 ml HCl per 500 mL of broth (resulting in a pH of 5).

Three plates were incubated in a micro-aerobic environment created in jars using, the Campygen^{™} 2.5 L (Oxoid) system, at 37°C, 25°C and 42°C respectively.

The other three plates were incubated at 37°C in an aerobic, anaerobic and 5% CO₂-supplemented atmosphere, respectively.

After six days of incubation, growth was evaluated by microscopic examination of the overlaying broth.

In a 5% CO₂-supplemented environment and an aerobic atmosphere, no growth was obtained. In an anaerobic environment, only very few bacteria were seen. In micro-aerobic conditions, good growth was seen at 37°C, while at 25°C or 42°C, no growth was obtained.

### Example 3: Effect of activated charcoal and growth factors on "Candidatus Helicobacter suis" growth.

Isolates HS1 and HS3 were inoculated on Mueller-Hinton agar plates, supplemented with Vitox supplement, Skirrow supplement, fungizone and 20 % foetal calf serum, adjusted to pH 5.0.

To these plates, the following components were added. One type of plates (MH1), in addition contained Vitox supplement but no activated charcoal. Another type of plates (MH2) in addition contained activated charcoal but no Vitox supplement, while a third type of plates (MH3) contained both Vitox supplement and activated charcoal.

After three and seven days of incubation at 37°C in micro-aerobic conditions in a closed circuit, created by evacuating 80% of the normal atmosphere and introducing a gas mixture of 8% CO₂, 8% H₂ and 84% N₂, growth was evaluated by microscopic examination of the overlaying broth.

Media MH1 and MH3 showed good growth of isolates HS1 and HS3. On MH2, no growth was seen for either of the isolates.

When using Mueller Hinton media, the omission of Vitox growth supplement impedes growth while the presence or absence of activated charcoal has no or little effect.

### Example 4: Effect of alternative nutrient compositions on "Candidatus Helicobacter suis" growth.

In the present experiment, Mueller-Hinton agar or Brucella agar was replaced by another medium for fastidious bacteria namely bovine Brain Heart Infusion (BHI) agar plates. These plates were supplemented with Vitox supplement, Skirrow supplement; fungizone and 20% foetal calf serum and were adjusted to pH 5 with concentrated HCl.

Plates were overlaid with BHI broth, supplemented with 20% foetal calf serum. After four days of incubation on these plates of isolates HS1 and HS2, at 37°C in a micro-aerobic environment, growth was confirmed by microscopic examination of the overlaying broth.

### Example 5: Effect of serum in plates on "Candidatus Helicobacter suis" growth.

Isolate HS1 was inoculated on a) Mueller-Hinton agar plates supplemented with Skirrow supplement, fungizone adjusted to pH 5.0 with concentrated HCl and on b) the same plates as in a) comprising in addition 20% FCS and vitox supplement.

Plates were overlaid with Mueller-Hinton broth, supplemented with Vitox supplement, Skirrow supplement, fungizone, 20% FCS and adjusted to pH 5.0 with concentrated HCl.

After three days of incubation at 37°C in a micro-aerobic environment on plates a) or b), growth was evaluated by microscopic examination of the broth. Coccoid forms only were observed on plates without serum in the agar (a). Good growth of HS1 was obtained on plates comprising the standard amount of 20% FCS (b).

Isolate HS1 was then inoculated on c) Mueller-Hinton agar plates supplemented with Skirrow supplement, fungizone adjusted to pH 5.0 with concentrated HCl, and overlaid with Mueller-Hinton broth, supplemented with Vitox supplement, Skirrow supplement, fungizone, 40% FCS and adjusted to pH 5.0 with concentrated HCl.

After four days of incubation at 37°C in a micro-aerobic environment on plates c), growth was evaluated by microscopic examination of the broth. Again, coccoid forms only were observed.

### Example 6: Effect of blood on "Candidatus Helicobacter suis" growth.

The use of blood instead of serum was evaluated by replacing 20 % FCS in the plates with 10% sheep or 10% horse blood. The pH of the plates was adjusted to pH 5.0 with concentrated HCl.

The plates were overlaid with Mueller-Hinton broth

After four days of incubation at 37°C in a micro-aerobic environment, growth was evaluated by microscopic examination of the overlaying broth.

Very good growth of both isolates was obtained, regardless of the origin of the blood used, indicating that blood can be added to the culture medium, instead of serum.

### Example 7: Effect of the concentration of blood on "Candidatus Helicobacter suis".

Isolate HS1 was inoculated on Mueller-Hinton agar plates supplemented with Vitox supplement, Skirrow supplement, fungizone and differing amounts of horse blood (2.5%, 5%, 7%, 10% and 15% (v/v)). The pH of the plates was adjusted to pH 5.0 with concentrated HCl.

Plates were overlaid with Mueller-Hinton broth without further supplements.

After three days of incubation at 37°C in a micro-aerobic environment, growth was evaluated by microscopic examination of the broth. On plates with 2.5%, 5% or 7% horse blood, only coccoid forms were seen. Growth of *"Candidatus* Helicobacter suis" was observed on the plates with 10% or 15% horse blood. Of the plates with 10 and 15 % blood, broth was then subcultured onto new agar plates with blood concentrations between 2.5 and 15 % containing the same medium, by inoculating 1 ml of culture and adding 1 ml of fresh broth. Plates were further incubated at the same conditions for another three days. Again viable cultures were only observed on plates with 10% or 15% horse blood.

### Example 8: Effect of the pH on "Candidatus Helicobacter suis" growth.

Isolate HS1 was inoculated on Mueller-Hinton agar plates supplemented with Vitox supplement, Skirrow supplement, fungizone and 20% FCS and adjusted to different pH values.

Plates were overlaid with Mueller-Hinton broth, supplemented with Vitox supplement, Skirrow supplement, fungizone and 20% FCS adjusted with concentrated HCl to different pH values. The specific combinations of pH of agar and broth are shown in Table 1.

After three days of incubation at 37°C in a micro-aerobic environment, growth was evaluated by microscopic examination of the broth. Broth was then subcultured onto new agar plates by inoculating 1 ml of the culture and adding 1 ml of fresh broth with the same pH value. Primary cultures and subcultures were evaluated after 2 days of incubation.

First subcultures were again transferred onto new agar plates as described above. Growth was evaluated after 4 and 7 days of incubation, respectively.

Second subcultures on media with pH 5.3 and 4.8 were transferred onto fresh agar plates. First subcultures on media with pH of 4.75, 4.5, 4.4 and 4 were also transferred onto fresh plates. Growth was evaluated after 3 and 7 days of incubation, respectively.

**Table 1. Growth of H. suis isolate HS1 after subculturing on Mueller-Hinton media with different pH values**

| | | **Primary culture** | | **first subculture** | | | **second subculture** | | **third subculture** | |
|---|---|---|---|---|---|---|---|---|---|---|
| **pH Agar** | **pH Broth** | 3 days | 5 days | 2 days | 4 days | 8 days | 4 days | 7 days | 3 days | 7 days |
| 5.0 | 7.0 | ++++ | ND | ND | ND | ND | ND | ND | ND | ND |
| 6.8 | 6.8 | +++ | ++ | ++ | + | - | ND | ND | ND | ND |
| 6.7 | 6.7 | ++ | ++ | ++ | +++ | + | - | +/- | ND | ND |
| 6.6 | 6.6 | ++ | + | ++ | +++ | + | - | - | ND | ND |
| 6.5 | 6.5 | ++ | + | ++ | +++ | + | + | - | ND | ND |
| 6.2 | 6.3 | ++ | ++++ | ++++ | +++ | ++ | + | - | ND | ND |
| 5.9 | 6.1 | +++ | ++++ | ++++ | +++ | ++ | ++ | + | ND | ND |
| 5.4 | 5.3 | +++ | ++++ | ++++ | +++ | +++ | ++++ | + | +++ | ND |
| 4.8 | 4.8 | +++ | ++++ | + | +++ | ++++ | ++++ | +++ | +++ | ND |
| 4.7 | 4.6 | +++ | ++ | + | + | ++ | ND | ND | ++++ | ++++ |
| 4.7 | 4.4 | ++ | ++ | + | + | ++ | ND | ND | + | + |
| 4.4 | 4.4 | + | + | + | + | ++ | ND | ND | ++ | - |
| 4.1 | 4.2 | + | + | + | + | ++ | ND | ND | + | - |
| 3.7 | 3.9 | - | - | + | - | - | ND | ND | ND | ND |
| 3.5 | 3.6 | - | - | + | - | - | ND | ND | ND | ND |

| | | | | | | | | | | |
|---|---|---|---|---|---|---|---|---|---|---|
| ND: Not Done; ++++ : very good growth; +++ : good growth; ++ : moderate growth; + : weak growth ; +/- very few bacteria; -: no growth. | | | | | | | | | | |

The isolate used (HS1) could not grow on media with a pH below 4 (pH 3.5 or 3.7). Primary cultures were obtained on media with pH values ranging from 4 to 7. Subculturing was successful on these plates, however, repeated subculturing was only successful on media with pH values ranging from 4.7 to 6. Growth of "*Candidatus* Helicobacter suis" is possible on media with a pH ranging from 4.5 to 6.0 with an optimum at pH values 4.7 and 5.5.

Summarising "*Candidatus* Helicobacter suis" has an optimal growth at 37°C in micro-aerobic conditions on a two component system. The solid component comprises a medium for fastidious micro-organisms (such as Brucella agar, Brain Heart Infusion agar or Mueller-Hinton agar), supplemented with Foetal calf serum or blood, growth supplements and adjusted to pH 5.0. Plates are overlaid with the liquid component which is a broth of a growth medium for fastidious micro-organisms (such as Brucella broth, Brain Heart Infusion broth or Mueller-Hinton broth) with or without growth supplements, serum or pH adjustment.

### Example 9: Vaccination of pigs using "Candidatus Helicobacter suis" antigens obtained from "Candidatus Helicobacter suis" isolates.

The safety and efficacy of the "*Candidatus* Helicobacter suis" vaccine is determined using a sonicated filtrate. The "*Candidatus* Helicobacter suis" antigen is inactivated and disrupted by sonication, followed by sterile filtration and formulation with an oil-in-water emulsion adjuvant. The antigen dose is determined based on the amount of total protein present.

### Study Design

Sows are selected from a herd preferably free of "*Candidatus* Helicobacter suis" infection, as determined by PCR and urease screening of stomachs from herdmates at slaughter. Piglets from these sows are then allotted to one of three groups: a saline control, "*Candidatus* Helicobacter suis" vaccine group, or a third group (NTX; untreated and fed a normal diet). The study is a completely randomized design, and each animal is the experimental unit. Following weaning, sows are euthanized and the stomach analyzed by PCR for the presence of *H. suis.* Piglets from any sow found PCR positive for *H. suis* in the stomach are excluded from the study.

**Table 2. Study Design**

| **Treatment** | **Vaccine** | **Regimen** | **Age at Vaccination^{#}** | **Challenge** |
|---|---|---|---|---|
| T01 | Saline | IM | 1,3,5 wk | 5,6,7,8 wk |
| T02 | *H. suis* | IM | 1,3,5 wk | 5,6,7,8 wk |
| NTX⁺ | NA | NA | NA | NA |

| | | | | |
|---|---|---|---|---|
| ^{#} Intramuscular (IM) injection in the neck, alternately left (wk1), right (wk 3), and then left (wk 5). All pigs will be euthanized at 14 weeks of age. ⁺ NTX= not treated; controls fed a normal diet during the trial. | | | | |

**Table 3. Antigen and IVP Preparation**

| **IVP*** | **Antigen Dose** | **Adjuvant^ per Dose** | **Volume per Dose** |
|---|---|---|---|
| Saline (PBS) | NA | 1 mL | 2 mL |
| *H. suis* | 250 µg first two doses; 500 µg third dose | 1 mL | 2 mL |

| | | | |
|---|---|---|---|
| * IVP = Investigational Veterinary Product ^ The adjuvant- a final concentration of 5% Amphigen- will be mixed 1:1 with the antigen. | | | |

### Vaccination and Challenge

One day before the first vaccination and at necropsy, each individual pig is weighed. Pigs are vaccinated by intramuscular injection in the neck at 1, 3, and 5 weeks of age. Control pigs are vaccinated at the same time with an equal volume of saline. Pigs are observed for clinical signs 1 day before and two days after vaccination, and within 1 hour post vaccination. On the day of the first vaccination, 2 weeks after the third vaccination, and just prior to necropsy, a blood sample (approximately 2 to 10 mL in serum separator tubes) is collected from each pig for *"Candidatus* Helicobacter suis" serology. Following separation, the serum is placed in at least 2 cryogenic vials. Serum samples are frozen at -20°C for subsequent analysis by ELISA for antibodies specific to *"Candidatus* Helicobacter suis".

For the H. suis challenge, homogenates of scrapings of the upper cell layers and mucus of the antrum of gastric samples which have been tested positive for H. suis (using urease test) is used. To confirm the viability and dose of the pig stomach homogenate used as challenge material, an aliquot of each challange material is administered to five week-old male SPF BALB/c mice free from H. suis infection. Mice are sacrificed 2 weeks later and the stomach contents screened by urease and PCR for the presence of H. suis.

### Post-Challenge Methods and Scoring

To determine infection, intestinal tracts are dissected and the mucosal surface from the pars oesophagea is macroscopically examined. Lesions are scored on a scale of 0-5 using the method of Hessing et al. (1992, Tijdschrift voor Diergeneeskunde 117, 445-450). Briefly, scores are recorded as follows: 0 = Intact mucosa; **1** = Mild hyperkeratosis (<50% surface area); 2 = Severe hyperkeratosis (≥ 50% of surface area); 3 = Hyperkeratosis and a few small erosions (less than 5 and shorter than 2.5 cm); 4 = Hyperkeratosis and extensive erosions (more than 5 erosions and/or longer than 2.5 cm); 5 = Hyperkeratosis and very large erosions (more than 10 erosions or longer than 5 cm) and/or ulcers. Each stomach is also scored using a visual analog scale from 0-100 mm where 0= No lesion and 100= Perforating ulcer.

After scoring, several sites from the glandular mucosa (approximately 0.5 cm²) from each stomach are sampled by PCR, quantitative urease test, and histology. PCR for specific detection *"Candidatus* Helicobacter suis" is performed as described by De Groote et al. (2000, above). The bacterial load is tested by the quantitative urease test described herein. For histopathological examination, gastric mucosal tissue samples are fixed in 10% phosphate buffered formalin, processed by routine methods, and embedded in paraffin. One 5 µm section is immunohistochemically stained with a polyclonal goat anti-*H. pylori* antibody (Dakocytomation, Denmark A/S: Glostrup, Denmark) as described by De Groote et al. (2000). A second section is stained with HE for scoring of gastritis. Histopathological changes are scored for 1) Diffuse lymphocytes - a score for the diffuse infiltration of lymphocytes in the propria mucosae; 2) Formation of lymphoid follicles in the propria mucosae; 3) Formation of lymphoid follicles under the surface epithelium; 4) Diffuse infiltration of plasma cells in the propria mucosae . The presence of lymphoid follicles under the surface epithelium and the infiltration of plasma cells are very noteworthy and characteristic lesions. These are scored by severity with a score of 0-3. 1 = mild, 2 = moderate, and 3= severe. They are also scored using a visual analog scale (0 -100 mm).

### Results

The mean urease values per antrum, fundus, or cardia of the stomach tissue of vaccinated pigs are significantly (P<0.10) less than for non-vaccinated pigs for the antrum, fundus, and/or cardia. The percentage of stomachs PCR positive for *"Candidatus* Helicobacter suis" are significantly (P<0.10) greater for non-vaccinated challenged pigs vs. vaccinated-challenged pigs. The stomachs of NTX pigs are negative as determined by PCR and urease.

The secondary variables are histological (lymphocytic deep and surface follicle formation and number of plasma cells) and gross gastric ulcer scores. It is expected that there will be a significantly (P≤0.10) greater mean lesion score in *"Candidatus* Helicobacter suis" non-vaccinated challenged pigs than vaccinated challenged pigs.

## Claims

1. A method for the isolation of a *Helicobacter* species from a sample, comprising the steps of cultivating said sample, comprising said *Helicobacter* species, in a cultivation system, said system comprising a cultivation medium having a pH between 5.0 and 6.0, and which medium is supplemented with at least 10 % serum or at least 7.5 % blood.

2. The method according to claim 1, wherein said cultivation medium comprises nutrients for the growth of fastidious bacteria.

3. The method according to claim 2, wherein said nutrients for the growth of fastidious bacteria are selected from the group consisting of Brucella, Mueller-Hinton or Brain Heart Infusion nutrients.

4. The method according to claim 1 or 2, wherein said cultivation medium comprises at least one selective substance that inhibits the growth of fungi and/or Gram positive and/or Gram negative micro-organisms other than the *Helicobacter* species to be cultured.

5. The method according to claim 4, wherein said at least one selective substance is selected from the group consisting of: vancomycin, trimethoprim lactate, polymyxin B, cefsulodin, colistin, amphotericin B, crystal violet, nystatin and nisin.

6. The method according to claim 1, wherein said cultivation medium comprises one or more growth factors selected from the group consisting of Vitamin B12, L-glutamine, Adenine, Guanine, p-Aminobenzoic acid, L-cystine, NAD (Coenzyme 1), Cocarboxylase, Ferric nitrate, Thiamine and Cysteine hydrochloride.

7. The method according to any one of claims 1 to 6, wherein said *Helicobacter* species is *Helicobacter suis.*

8. The method according to claim 7, wherein the 16S rRNA gene sequence of said *Helicobacter* species has at least 99 % sequence identity with the 16S rRNA gene sequence of Genbank accession number AF127028 of November 17, 1999 depicted in SEQ ID NO:7 and Figure 1.

9. A *Helicobacter suis* isolate, wherein the 16S rRNA gene sequence of *Helicobacter suis* has at least 99 % sequence identity with the 16S rRNA gene sequence of Genbank accession number AF127028 of November 17, 1999 depicted in SEQ ID NO:7 and Figure 1, free from bacteria or fungi present in the stomach, obtainable by the methods of any one of claims 1 to 8.

10. The method according to any one of claims 1 to 8 further comprising the step of passaging a *Helicobacter* species as isolated by a method according to any one of claims 1 to 8, comprising the step of applying said isolated *Helicobacter* species in a cultivation system, said system comprising a cultivation medium having a pH between 4.0 and 7.0, which medium is supplemented with at least 10 % serum or at least 7.5 % blood, and incubating the isolated *Helicobacter* species therein.

11. The method according to claim 10, wherein said *Helicobacter* species is *Helicobacter suis.*

12. Use of a cultivation recipient comprising a solid cultivation medium, the solid medium comprising blood in a concentration between 7.5 % and 15 % or comprising serum in a concentration between 12.5 % and 25 %, wherein the pH of said solid medium is between 5.0 and 5.5, for the isolation and/or cultivation of a *Helicobacter* species,

13. A vaccine comprising an antigen preparation of a *Helicobacter suis* isolate, wherein the antigen preparation is selected from the group consisting of :
- whole-killed *Helicobacter suis* bacteria,
- live-attenuated *Helicobacter suis* weakened bacteria, and
- a lysate of whole-killed inactive *Helicobacter suis* bacteria or live-attenuated weakened *Helicobacter suis* bacteria,
- the soluble fraction of a sonicated bacterial culture of *Helicobacter suis,* and
- a protein of *Helicobacter suis* selected from the group consisting of a heat shock protein, a urease protein, cagA and vacA,
wherein said *Helicobacter suis* has a 16S rRNA gene sequence which is at least 99 % identical with the 16S rRNA gene sequence of Genbank accession number AF127028 of November 17, 1999 depicted in SEQ ID NO:7 and Figure 1.

14. A method for producing an antigen preparation of *Helicobacter suis,* said method comprising the steps of:
isolating *Helicobacter suis* according to the method according to any one of claims 1 to 8 or claims 10 or 11, and
producing an antigen preparation from the obtained *Helicobacter suis* isolate.

15. Use of one or more of:
- a lysate of a *Helicobacter suis* isolate,
- the soluble fraction of a sonicated bacterial culture of *Helicobacter suis,*
- a protein of *Helicobacter suis* selected from the group consisting of a heat shock protein, a urease protein, cagA and vacA,
- a live-attenuated *Helicobacter suis* isolate, and
- a whole-killed *Helicobacter suis* isolate,
for the manufacture of a vaccine against a *Helicobacter suis* infection,
wherein said *Helicobacter suis* has a 16S rRNA gene sequence of which is at least 99 % identical with the 16S rRNA gene sequence of Genbank accession number AF127028 of November 17, 1999 depicted in SEQ ID NO:7 and Figure 1.

## Patentansprüche

1. Verfahren zum Isolieren einer *Helicobacter* Spezies aus einer Probe, umfassend die Schritte des Kultivierens der Probe, die die *Helicobacter* Spezies enthält, in einem Kultivierungssystem, wobei das System ein Kultivierungsmedium mit einem pH zwischen 5,0 und 6,0 umfasst und wobei das Medium mit mindestens 10% Serum oder mindestens 7,5% Blut ergänzt ist.

2. Verfahren nach Anspruch 1, wobei das Kultivierungsmedium Nährstoffe für das Wachstum anspruchsvollerer Bakterien enthält.

3. Verfahren nach Anspruch 2, wobei die Nährstoffe für das Wachstum anspruchsvollerer Bakterien ausgewählt sind aus der Gruppe von Brucella, Mueller-Hinton oder Hirn-Herz Infusionsnährstoffen.

4. Verfahren nach Anspruch 1 oder 2, wobei das Kultivierungsmedium mindestens eine selektive Substanz enthält, die das Wachstum von Pilzen und/oder grampositiver und/oder gramnegativer Mikroorganismen hemmt, die nicht die zu kultivierende *Helicobacter* Spezies sind.

5. Verfahren nach Anspruch 4, wobei die mindestens eine selektive Substanz ausgewählt ist aus der Gruppe bestehend aus: Vancomycin, Trimethoprimlactat, Polymyxin B, Cefsulodin, Colistin, Amphotericin B, Kristallviolett, Nystatin und Nisin.

6. Verfahren nach Anspruch 1, wobei das Kultivierungsmedium einen oder mehrere Wachstumsfaktoren enthält, die ausgewählt sind aus der Gruppe bestehend aus Vitamin B12, L-Glutamin, Adenin, Guanin, p-Aminobenzoesäure, L-Cystin, NAD (Co-Enzym 1), Cocarboxylase, Eisennitrat, Thiamin und Cysteinhydrochlorid.

7. Verfahren nach einem der Ansprüche 1 bis 6, wobei die *Helicobacter* Spezies *Helicobacter suis* ist.

8. Verfahren nach Anspruch 7, wobei die 16S rRNA Gensequenz der *Helicobacter* Spezies mindestens 99% Sequenzidentität mit der 16S rRNA Gensequenz der Genbank-Zugangsnummer AF127028 vom 7. November 1999 hat, die in SEQ ID NR:7 und Figur 1 dargestellt ist.

9. *Helicobacter suis* Isolat, wobei die 16S rRNA Gensequenz von *Helicobacter suis* mindestens 99% Sequenzidentität mit der 16S rRNA Gensequenz von Genbank-Zugangsnummer AF127028 vom 7. November 1999 hat, die in SEQ ID NR:7 und Figur 1 dargestellt ist, frei von Bakterien oder Pilzen ist, die im Magen vorhanden sind, erhältlich durch die Verfahren nach einem der Ansprüche 1 bis 8.

10. Verfahren nach einem der Ansprüche 1 bis 8, des Weiteren umfassend den Schritt des Passagierens einer *Helicobacter* Spezies, wie durch ein Verfahren nach einem der Ansprüche 1 bis 8 isoliert, umfassend den Schritt des Anwendens der isolierten *Helicobacter* Spezies in einem Kultivierungssystem, wobei das System ein Kultivierungsmedium mit einem pH zwischen 4,0 und 7,0 enthält, wobei das Medium mit mindestens 10% Serum oder mindestens 7,5% Blut ergänzt ist, und Inkubieren der isolierten *Helicobacter* Spezies darin.

11. Verfahren nach Anspruch 10, wobei die *Helicobacter* Spezies *Helicobacter suis* ist.

12. Verwendung eines Kultivierungsempfängers, umfassend ein festes Kultivierungsmedium, wobei das feste Medium Blut in einer Konzentration zwischen 7,5% und 15% enthält oder Serum in einer Konzentration zwischen 12,5% und 25% enthält, wobei der pH des festen Medium zwischen 5,0 und 5,5 beträgt, zur Isolierung und/oder Kultivierung einer *Helicobacter* Spezies,

13. Impfstoff, umfassend eine Antigenzubereitung eines *Helicobacter suis* Isolats, wobei die Antigenzubereitung ausgewählt ist aus der Gruppe bestehend aus:
- vollständigen abgetöteten *Helicobacter suis* Bakterien,
- lebend-attenuierten *Helicobacter suis* abgeschwächten Bakterien, und
- einem Lysat aus vollständigen abgetöteten inaktiven *Helicobacter suis* Bakterien oder lebend-attenuierten abgeschwächten *Helicobacter suis* Bakterien,
- der löslichen Fraktion einer beschallten Bakterienkultur von *Helicobacter suis,* und
- einem Protein von *Helicobacter suis,* ausgewählt aus der Gruppe bestehend aus einem Wärmeschockprotein, einem Ureaseprotein, cagA und vacA,
wobei *Helicobacter suis* eine 16S rRNA Gensequenz hat, die mindestens 99% mit der 16S rRNA Gensequenz von Genbank-Zugangsnummer AF127028 vom 7. November 1999, die in SEQ ID NR:7 und Figur 1 dargestellt ist, identisch ist.

14. Verfahren zur Herstellung einer Antigenzubereitung von *Helicobacter suis,* wobei das Verfahren folgende Schritte umfasst:
Isolieren von *Helicobacter suis* nach dem Verfahren nach einem der Ansprüche 1 bis 8 oder Ansprüchen 10 oder 11, und
Herstellen einer Antigenzubereitung aus dem erhaltenen *Helicobacter suis* Isolat.

15. Verwendung von einem oder mehreren von:
- einem Lysat eines *Helicobacter suis* Isolats,
- der löslichen Fraktion einer beschallten Bakterienkultur von *Helicobacter suis,*
- einem Protein von *Helicobacter suis,* ausgewählt aus der Gruppe bestehend aus einem Wärmeschockprotein, einem Ureaseprotein, cagA und vacA,
- einem lebend-attenuierten *Helicobacter suis* Isolat und
- einem vollständigen-abgetöteten *Helicobacter suis* Isolat,
für die Herstellung eines Impfstoffs gegen eine *Helicobacter suis* Infektion, wobei *Helicobacter suis* eine 16S rRNA Gensequenz hat, die mindestens 99% mit der 16S rRNA Gensequenz von Genbank Zugangsnummer AF127028 vom 7. November 1999, die in SEQ ID NR:7 und Figur 1 dargestellt ist, identisch ist.

## Revendications

1. Une méthode pour l'isolement d'une espèce *Helicobacter* à partir d'un échantillon, comprenant les étapes consistant à cultiver ledit échantillon, comprenant ladite espèce d'*Helicobacter*, dans un système de culture, ledit système comprenant un milieu de culture ayant un pH entre 5,0 and 6,0, et dans lequel ledit milieu est additionné avec au moins 10 % de sérum ou au moins 7,5 % de sang.

2. La méthode selon la revendication 1, dans laquelle ledit milieu de culture comprend des agents nourissants pour la croissance de bactéries fastidieuses.

3. La méthode selon la revendication 2, dans laquelle lesdits agents nourissants pour la croissance de bactéries fastidieuses sont choisis parmi le groupe constitué des agents nourissants Brucella, Mueller-Hinton ou Infusion Cerveau Coeur.

4. La méthode selon la revendication 1 ou 2, dans laquelle ledit milieu de culture comprend au moins une substance sélective qui inhibe la croissance de champignons et / ou de micro-organismes Gram positifs et / ou Gram négatifs autres que l'espèce *Helicobacter* devant être cultivée.

5. La méthode selon la revendication 4, dans laquelle ladite au moins une substance sélective est choisie parmi le groupe constitué de: vancomycine, triméthoprim lactate, polymyxine B, cefsulodine, colistine, amphotéricine B, cristal violet, nystatine et nisine.

6. La méthode selon la revendication 1, dans laquelle ledit milieu de culture comprend un ou plusieurs facteurs de croissance choisis parmi le groupe constitué de la vitamine B12, L-glutamine, Adénine, Guanine, acide p-aminobenzoïque, L-cystine, NAD (co-enzyme 1), cocarboxylase, nitrate ferrique, thiamine et chlorhydrate de cysteine.

7. La méthode selon l'une quelconque des revendications 1 à 6, dans laquelle ladite espèce *Helicobacter* est *Helicobacter suis.*

8. La méthode selon la revendication 7, dans laquelle la séquence de gène 16S rRNA de ladite espèce *Helicobacter* possède au moins 99 % d'identité de séquence avec la séquence de gène 16S rRNA de Genbank numéro d'accès AF127028 du 17 novembre 1999 décrite dans SEQ ID NO:7 et la figure 1.

9. Un isolat d'*Helicobacter* suis, dans laquelle la séquence de gène 16S rRNA de *Helicobacter suis* possède au moins 99 % d'identité de séquence avec la séquence de gène 16S rRNA de Genbank numéro d'accès AF127028 du 17 novembre 1999 décrite dans SEQ ID NO:7 et la figure 1, exempt de bactéries ou de champignons présents dans l'estomac, et pouvant être obtenu par les méthodes de l'une quelconque des revendications 1 à 8.

10. La méthode selon l'une quelconque des revendications 1 à 8, comprenant en outre l'étape consistant à repiquer une espèce *Helicobacter* telle qu'isolée par une méthode selon l'une quelconque des revendications 1 à 8, comprenant l'étape consistant à appliquer ladite espèce *Helicobacter* isolée dans un système de culture, ledit système comprenant un milieu de culture ayant un pH entre 4,0 et 7,0, dans lequel ledit milieu est additionné avec au moins 10 % de sérum ou au moins 7,5 % de sang, et à incuber l'espèce *Helicobacter* isolée dans ce milieu.

11. La méthode selon la revendication 10, dans laquelle ladite espèce *Helicobacter* est *Helicobacter suis.*

12. Utilisation d'un récipient de culture comprenant un milieu de culture solide, le milieu solide comprenant du sang dans une concentration entre 7,5 % et 15 % ou bien comprenant du sérum dans une concentration entre 12,5 % et 25 %, dans laquelle le pH dudit milieu solide est entre 5,0 et 5,5, pour l'isolement et/ou la culture d'une espèce *Helicobacter.*

13. Un vaccin comprenant une préparation d'antigène d'un isolat de *Helicobacter suis,* dans lequel la préparation d'antigène est choisir parmi le groupe constitué de :
- bactéries *Helicobacter suis* entièrement inactivées,
- des bactéries *Helicobacter suis* vivantes atténuées affaiblies,
- un lysat de bactéries *Helicobacter suis* inactives entièrement tuées ou bien de bactéries *Helicobacter suis* vivantes atténuées affaiblies,
- la fraction soluble d'une culture de bactéries *Helicobacter suis* soumise à ultrasons, et
- une protéine de *Helicobacter suis* choisie parmi le groupe constitué d'une protéine du choc thermique, une protéine d'uréase, cagA et vacA,
dans lequel ledit *Helicobacter suis* possède une séquence de gène 16S rRNA qui est au moins 99 % identique avec la séquence de gène 16S rRNA de Genbank numéro d'accès AF127028 du 17 novembre 1999 décrite dans SEQ ID NO:7 et la figure 1.

14. Une méthode pour produire une préparation d'antigène de *Helicobacter suis*, ladite méthode comprenant les étapes consistant à:
isoler *Helicobacter suis* en conformité avec la méthode selon l'une quelconque des revendications 1 à 8 ou des revendications 10 ou 11, et
produire une préparation d'antigène à partir de l'isolat de *Helicobacter suis* obtenu.

15. Utilisation d'un ou plusieurs de:
- un lysat d'un isolat de *Helicobacter suis,*
- la fraction soluble d'une culture de bactéries *Helicobacter suis* soumise à ultrasons,
- une protéine de *Helicobacter suis* choisie parmi le groupe constitué d'une protéine du choc thermique, une protéine d'uréase, cagA et vacA,
- un isolat de *Helicobacter suis* atténué vivant, et
- un isolat de *Helicobacter suis* entièrement tué,
pour la fabrication d'un vaccin contre une infection de *Helicobacter suis,* dans laquelle ledit *Helicobacter suis* possède une séquence de gène 16S rRNA qui est au moins 99 % identique avec la séquence de gène 16S rRNA de Genbank numéro d'accès AF127028 du 17 novembre 1999 décrite dans SEQ ID NO:7 et la figure 1.
